(19) Europäisches Patentamt
European Patent Office
Office européen des brevets

(11) Veröffentlichungsnummer: **0 251 308 B1**

# (12) EUROPÄISCHE PATENTSCHRIFT

(45) Veröffentlichungstag der Patentschrift: **22.11.90**

(21) Anmeldenummer: **87109461.1**

(22) Anmeldetag: **01.07.87**

(51) Int. Cl.⁵: **C07D 487/08**, C07D 519/00, A61K 31/47 // (C07D487/08, 209:00, 209:00),(C07D487/08, 241:00, 209:00)

(54) **4-Chinolon-3-carbonsäurederivate, Verfahren zu ihrer Herstellung und diese enthaltende pharmazeutische Präparate.**

(30) Priorität: **04.07.86 DE 3622509**

(43) Veröffentlichungstag der Anmeldung: **07.01.88 Patentblatt 88/1**

(45) Bekanntmachung des Hinweises auf die Patenterteilung: **22.11.90 Patentblatt 90/47**

(84) Benannte Vertragsstaaten: **AT BE CH DE ES FR GB GR IT LI LU NL SE**

(56) Entgegenhaltungen:
**EP-A- 0 058 392**
**EP-A- 0 090 424**
**EP-A- 0 181 521**
**EP-A- 0 215 650**
**US-A- 4 571 396**

**Die Akte enthält technische Angaben, die nach dem Eingang der Anmeldung eingereicht wurden und die nicht in dieser Patentschrift enthalten sind.**

(73) Patentinhaber: **Chemie Linz Gesellschaft m.b.H., St.Peter-Strasse 25, A-4021 Linz(AT)**

(84) Benannte Vertragsstaaten: **BE CH DE ES FR GB GR IT LI LU NL SE AT**

(72) Erfinder: **Sauter, Fritz, Dr., Zollergasse 2, A-1070 Wien(AT)**
Erfinder: **Jordis, Ulrich, Dipl.-Ing. Dr., Hofzeile 6, A-1190 Wien(AT)**
Erfinder: **Rudolf, Manfred, Dipl.-Ing. Dr., Gudrunstrasse 55-103/12/4, A-1100 Wien(AT)**
Erfinder: **Wieser, Josef, Dr., Boschweg 1c, A-4020 Linz(AT)**
Erfinder: **Baumann, Karl, Dr., Boschweg 1b, A-4020 Linz(AT)**

(74) Vertreter: **Kunz, Ekkehard, Dr., Chemie Holding AG Patentwesen St. Peter-Strasse 25, A-4021 Linz(AT)**

**EP 0 251 308 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue 4-Pyridonderivate, Verfahren zu ihrer Herstellung, pharmazeutische Präparate, die diese Verbindungen enthalten und ihre Verwendung als Wirkstoffe in Arzneimitteln zur Behandlung von bakteriellen Infektionen.

4-Pyridonderivate und hier insbesondere Oxoderivate von substituierten Chinolin-, Naphthyridin-, Benzoxazin- und Benzochinolizincarbonsäuren mit antibakterieller Wirkung sind bekannt. Aus dieser Verbindungsklasse sind zum Beispiel in der US-PS 4 146 719 Derivate der Chinolon-3-carbonsäure beschrieben, die als besonderes Strukturelement eine cyclische Stickstoffbase in 7-Stellung des Chinolonrings aufweisen und antibakterielle Aktivität besitzen.

In der EP 131 839 werden u.a. antibakteriell wirksame Chinoloncarbonsäurederivate beschrieben, die in der 1-Stellung mit p-Fluorphenyl substituiert sein können und die als charakteristischen Substituenten in 7-Stellung eine aliphatische, ringförmige heterocyclische Gruppe tragen können. Überbrückte, bicyclische Basen werden als Substituenten jedoch nicht erwähnt.

Aus der EP-A 159 174 sind Oxoderivate von substituierten Chinolin-, Naphthyridin-und Benzoxazincarbonsäuren bekannt, die als charakteristischen Substituenten in der 7-Stellung des Chinolin- oder Naphthyridinringes bzw. in der 10-Stellung des Benzoxazinringes eine überbrückte, bicyclische Stickstoffbase tragen und ebenfalls antibakteriell wirksam sind.

In der EP-A 215 650 werden antibakteriell wirksame Chinoloncarbonsäuren beschrieben, die in Position 1 unter anderem durch 4-Fluorphenyl und in Position 7 durch eine überbrückte, bicyclische Stickstoffbase substituiert sind.

Trotz der großen Zahl der in der Literatur beschriebenen, antibakteriell wirksamen 4-Pyridonderivate besteht noch immer ein Bedarf für neue Verbindungen aus dieser Substanzklasse, da bekannte 4-Pyridonderivate hinsichtlich ihrer Aktivität, Wirksamkeit gegen bestimmte Typen von bakteriellen Erregern, Stabilität, pharmakokinetischen Eigenschaften und/oder toxischer Nebenwirkungen verbessert werden können.

Es wurden nun neue, mit überbrückten Stickstoffbasen substituierte Oxoderivate von besonderen Chinolin- und Benzoxazincarbonsäuren gefunden, die eine gegenüber den bekannten Verbindungen verbesserte pharmakologische Wirkung aufweisen.

Gegenstand der vorliegenden Erfindung sind neue 4-Pyridonderivate der allgemeinen Formel

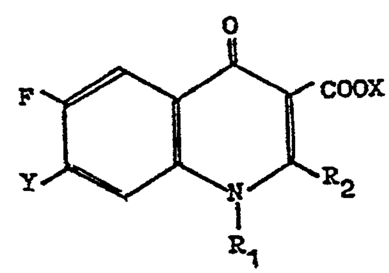

I

in der
$R_1$ 2,4-Difluorphenyl,
$R_2$ Wasserstoff
X Wasserstoff oder eine physiologisch unbedenkliche Estergruppe und
Y eine Base der allgemeinen Formel

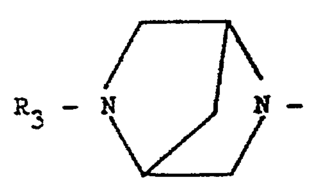

IIa

oder

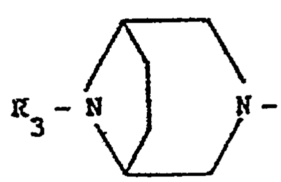

IIb

worin
$R^3$ für Wasserstoff, ($C_1$–$C_4$)-Alkyl, Benzyl, Formyl, Acetonyl oder einen Rest der allgemeinen Formel

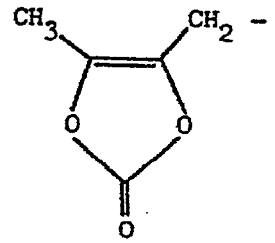

III

steht,
bedeuten, sowie Additionssalze von Verbindungen der allgemeinen Formel I mit pharmazeutisch unbedenklichen anorganischen oder organischen Säuren oder Basen und/oder deren Hydrate.

In der vorliegenden Beschreibung ist unter dem Begriff «($C_1$–$C_4$)-Alkyl» ein geradkettiger oder verzweigter gesättigter Kohlenwasserstoffrest mit 1 bis 4 Kohlenstoffatomen wie Methyl, Ethyl, Propyl, Isopropyl, Butyl, Isobutyl, sek. Butyl oder tert. Butyl zu verstehen. Der Begriff «physiologisch unbedenkliche Estergruppe» umfaßt auch Ester, welche die Resorption des Wirkstoffes erhöhen können, wie z. B. Pivaloyloxymethyl- oder 1-Acetoxyethylester.

Von den Verbindungen der Formel I sind solche bevorzugt, in denen $R_1$ 2,4-Difluorphenyl, Y eine Base der allgemeinen Formel IIa, in der $R_3$ Wasserstoff, Methyl oder Formyl darstellt, bedeutet.

Besonders bevorzugt sind die Verbindungen 6-Fluor-1-(2,4-difluorphenyl)-1,4-dihydro-7-(5-methyl-2,5-diaza-bicyclo(2.2.1)hept-2-yl)4-oxo-chinolin-3-carbonsäure und 7-(2,5-Diazabicyclo(2.2.1)hept-2-yl)-6-fluor-1-(2,4-difluorphenyl)-1,4-dihydro-4-oxo-chinolin-3-carbonsäure.

Die Verbindungen der vorliegenden Erfindung besitzen teilweise optische Zentren und können als Racemate oder in Form ihrer optischen Isomeren vorliegen. Die Erfindung umfaßt sowohl die Racemate als auch die optischen Isomeren.

Die erfindungsgemäßen Verbindungen der allgemeinen Formel I können dadurch hergestellt werden, dass man

a) Verbindungen der allgemeinen Formel

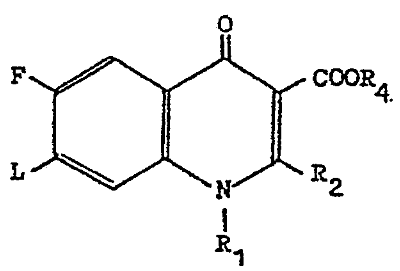

IV

in der $R_1$ und $R_2$ die in der allgemeinen Formel I angegebene Bedeutung haben, L eine nucleophile Abgangsgruppe wie Chlor oder Fluor, und $R_4$ Wasserstoff, ($C_1$–$C_4$)-Alkyl oder einen Borkomplex $-B(R_5R_6)$ darstellt, worin $R_5$ und $R_6$ gleich sind und Fluor oder einen Rest $-O(CO)$-($C_1$–$C_4$)-Alkyl bedeuten, mit Verbindungen der Formel

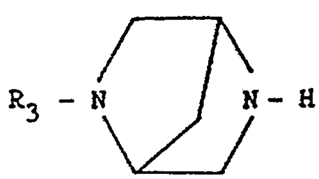

Va

oder

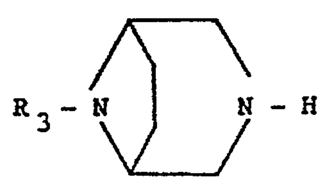

Vb

in der $R_3$ die in Formel I angegebene Bedeutung hat oder zusätzlich eine Aminoschutzgruppe bedeutet, umsetzt, worauf man

b) so erhaltene Verbindungen, in denen $R_4$ ($C_1$–$C_4$)-Alkyl oder die Gruppe $-B(R_5R_6)$ darstellt, durch Abspaltung von $-B(R_5R_6)$ oder von Alkyl in Verbindungen der Formel I, in denen X Wasserstoff bedeutet, überführt,

c) in erhaltenen Verbindungen, die in Position $R_3$ eine Aminoschutzgruppe tragen, diese abspaltet,

d) gegebenenfalls erhaltene Verbindungen der allgemeinen Formel I, in der $R_3$ Wasserstoff bedeutet,

methyliert, formyliert oder mit Bromaceton oder einer Verbindung $R_7$Hal, in der $R_7$ den Rest der Formel III bedeutet, umsetzt und

e) gewünschtenfalls in Stufen a) bis d) erhaltene Verbindungen der allgemeinen Formel I in ihre pharmazeutisch verträglichen Additionssalze mit Säuren oder Basen und/oder in ihre physiologisch unbedenklichen Ester überführt.

Bei der Umsetzung von Verbindungen der allgemeinen Formel IV mit Verbindungen der Formel V nach Verfahrensschritt a) verwendet man Basen als HX-Acceptoren. Als solche können z.B. im Überschuss eingesetzte Basen der allgemeinen Formel Va oder Vb verwendet werden. Hierbei setzt man auf 1 Mol der Verbindungen der allgemeinen Formel IV 1–20 Mol, vorzugsweise 1–5 Mol der Verbindungen der Formel Va oder Vb ein. Die im Überschuss eingesetzte Base der allgemeinen Formel Va oder Vb kann anschließend durch Aufarbeitung der Mutterlaugen rückgewonnen werden. Bei Verwendung äquivalenter Mengen der Verbindungen der allgemeinen Formel IV und der Basen der allgemeinen Formel Va oder Vb setzt man als säurebindende Mittel beispielsweise 1,4 Diazabicyclo(2,2,2)octan, Triethylamin oder 1,8-Diazabicyclo(5,4,0)undec-7-en ein.

Unter der nucleophilen Abgangsgruppe L der Formel IV ist eine Gruppe zu verstehen, die bei Reaktion einer Verbindung der Formel IV mit einer Verbindung der Formeln Va oder Vb unter Mitnahme der Bindungselektronen abgelöst wird. Insbesondere hat sie die Bedeutung von Fluor oder Chlor.

Die Umsetzung nach Verfahrensschritt a) wird vorteilhafterweise in Gegenwart von Lösungs- oder Verdünnungsmitteln wie beispielsweise Dimethylsulfoxid, 1,3-Dimethyl-tetrahydro-2(1H)-pyrimidinon, Hexamethylphosphorsäuretriamid, Sulfolan, Pyridin, Lutidin oder Collidin oder in Mischungen dieser Lösungsmittel durchgeführt. Es ist jedoch auch möglich, als Lösungsmittel eine der oben genannten Basen oder Mischungen dieser Basen mit einem der oben genannten Lösungsmittel zu verwenden.

Die Reaktionsdauer der Reaktion des Verfahrensschrittes a) beträgt in der Regel 1 bis 5 Stunden, die Reaktionstemperatur 100–200°C, wobei 130 bis 170°C bevorzugt sind.

Falls nach der Durchführung der Reaktion das Produkt beim Erkalten nicht auskristallisiert und durch einfache Filtration gewonnen werden kann, erfolgt die Isolierung wahlweise durch Abdestillieren der flüchtigen Anteile oder durch Ausfällen des Produktes oder eines seiner Salze, oder durch eine Kombination dieser Maßnahmen.

In einer besonders bevorzugten Variante des Verfahrensschrittes a) werden zur Erhöhung der Reaktivität solche Verbindungen der allgemeinen Formel IV eingesetzt, in denen $R_4$ die Bedeutung von $-B(R_5R_6)$ hat. Die Reaktionsdauer beträgt bei Verwendung dieser Borkomplexe im allgemeinen 1–30 Stunden bei einer Temperatur von Raumtemperatur bis 50°C.

Falls in Verfahrensschritt a) Verbindungen der Formel Va oder Vb eingesetzt werden, in denen $R_3$ die Bedeutung von Wasserstoff hat, ist es nicht notwendig, aber in vielen Fällen vorteilhaft, die Verbindungen der Formel Va oder Vb vor dieser Umsetzung durch Aminoschutzgruppen gegen eine nucleophile Substitution inert zu machen. Auf diese Weise können z.B. folgende Schutzgruppen Verwendung finden:

Carboxylsäuregruppen wie z.B. Formyl, Acetyl, Trifluoracetyl; Alkoxycarbonylgruppen wie z.B. Ethoxycarbonyl, t-Butoxycarbonyl, 2,2,2-Trichlorethoxycarbonyl; Aryloxycarbonylgruppen wie z.B. Benzyloxycarbonyl, Phenoxycarbonyl; und Gruppen wie z.B. Trimethylsilyl, Trityl, p-Toluolsulfonyl und Benzyl.

Die dem Verfahrensschritt b) entsprechende Abspaltung von $R_4$ mit der Bedeutung von $(C_1–C_4)$-Alkyl oder $-B(R_5R_6)$ erfolgt durch einstündiges Erhitzen unter Rückfluß des unter a) erhaltenen Produktes in einem ca. zehnfachen Überschuß von verdünnter Alkalihydroxidlösung. Nach dem Erhitzen wird die Lösung mit HCl neutralisiert, der ausgefallene Niederschlag abgesaugt und nach üblichen Methoden wie Waschen oder Umkristallisieren aufgearbeitet.

Die Abspaltung der Aminoschutzgruppe nach Verfahrensschritt c) kann nach bekannten Methoden vor oder nach der Isolierung des Produktes der Formel I erfolgen. So wird z.B. die Ethoxycarbonylgruppe durch Hydrolyse im sauren oder alkalischen Milieu abgespalten, die Tritylgruppe kann durch Hydrogenolyse entfernt werden.

Die Methylierung von Verbindungen der Formel I, in der $R_3$ die Bedeutung von Wasserstoff hat, nach Verfahrensschritt d) kann mit Hilfe üblicher Methylierungsmittel wie Methyljodid oder Dimethylsulfat oder durch Erhitzen des sekundären Amins unter Rückfluß in einem etwa 10–20 fachen Überschuß einer Mischung aus Ameisensäure und 40%-iger Formaldehydlösung erfolgen. Die Reaktionszeit beträgt etwa 0,5–5 Stunden. Das Reaktionsprodukt wird anschließend eingedampft, in Wasser aufgenommen, mit verdünntem Ammoniak auf pH 7 gebracht und mit einer Mischung aus Methylenchlorid und Chloroform mehrmals extrahiert. Eindampfen der vereinigten organischen Phasen und Umkristallisieren, beispielsweise aus Methanol/Ether ergibt das gewünschte alkylierte Produkt.

Die Formylierung von Verbindungen der Formel I, in der $R_3$ die Bedeutung von Wasserstoff hat, erfolgt mit Hilfe üblicher Formylierungsmittel wie beispielsweise des gemischten Anhydrides von Essigsäure und Ameisensäure. Der Umsatz mit Bromaceton oder einer Verbindung $R_7$Hal kann beispielsweise in einem inerten Verdünnungsmittel wie Dimethylformamid unter Zusatz von Säurebindern wie Kaliumhydrogencarbonat erfolgen.

Erwünschtenfalls führt man in einem Reaktionsschritt e) die nach a) bis d) erhaltenen Verbindungen, die amphoter sind, mit Basen oder Säuren in ihre Salze und/oder in ihre physiologisch verträglichen Ester über. Als Salze, die durch Reaktion von Basen mit der Carboxylgruppe der erfindungsgemäßen Verbindungen erhalten werden, kommen beispielsweise die Alkali-, Erdalkali-, Ammoniak- und Silbersalze sowie die Salze mit pharmazeutisch verträglichen Basen wie Cholin, Diethanolamin, Ethylendiamin, Guanidin u.ä. in Frage. Salze mit pharmazeutisch verträglichen anorganischen oder organischen Säuren werden durch Reaktion am basischen Zentrum gebildet. Beispiele solcher Säuren sind Salzsäure, Schwefelsäure, Phosphorsaure, Essigsäure, Zitronensäure, Oxalsäure, Malonsäure, Salicylsäure, Methansulfonsäure u.ä. Die Bildung der physiologisch verträglichen Ester kann nach allgemein bekannten Methoden erfolgen.

Als Ausgangsverbindungen benötigte Substanzen der Strukturformeln IV sind bekannt oder können analog zu bekannten Verfahren hergestellt werden, wie sie beispielsweise in der EP 47 005, EP 159 174, EP 203, EP 78 362 und in J. Med. Chem. 1980, 23, 1358 oder J. Med. Chem. 1985, 28, 1558 beschrieben sind. Die 6,1 Difluor-1-(4-fluorphenyl)-1,4-dihydro-4-oxo-chinolin-3-carbonsäure wurde nach Formelschema 11 hergestellt. Das Säurechlorid VI wurde mit dem Magnesiumsalz des Malonsäure-ethyl-tert-butylesters zum Acylmalonester VII umgesetzt, der durch Behandeln mit Trifluoressigsäure in den Aroylessigsäureethylester VIII überführt wurde. Durch Kondensation von VIII mit o-Ameisensäure-triethylester/Acetanhydrid wurde der 2-(2-Chlor-4,5-difluor-benzoyl)-3-ethoxy-acrylsäureethylester IX erhalten, der mit 4-Fluor-anilin zum Enamin X umgesetzt wurde. Das Enamin X wurde mit Natriumhydrid als Base zum Chinolonester XI (R = Ethyl) cyclisiert, der dann zur gewünschten Säure XI (R = H) verseift wurde.

Das als Ausgangsmaterial für diesen Syntheseweg verwendete 2-Chlor-4,5-difluorbenzoylchlorid VI wurde aus der entsprechenden Säure mit Thionylchlorid erhalten. Die 2-Chlor-4,5-difluorbenzoesäure wurde durch Samdmeyer-Reaktion aus der nach EP 55 629 hergestellten 4,5-Difluoranthranilsäure erhalten.

Die Herstellung der Borkomplexe der allgemeinen Formel IV ist ebenfalls literaturbekannt und kann z.B. nach den in den japanischen Anmeldungen 82 233 684, 83 188 138 und 85 126 290 beschriebenen Methoden erfolgen.

Die Basen der allgemeinen Formel V sind literaturbekannt und können gemäß den folgenden Literaturstellen oder leichten Abwandlungen hiervon hergestellt werden:

1) P.A. Sturm, D.W. Henry, P.F. Thompson, J.B. Zeigler und J.W. McCall J. Med.Chem. 1974, 17, 481
2) R.A. Barnes und H.M. Fales, J.Am. Chem.Soc. 1953, 75, 975
3) R.J. Michaels und H.E. Zaugg, J.Org. Chem. 1960, 25, 637
4) H. Newman J., Heterocycl. Chem. 1974, 11, 449
5) P.S. Portoghese und A.A. Mikhail, J.Org. Chem. 1966 31, 1059
6) P.C. Ruenitz und E.E. Smissman, J.Heterocycl .Chem. 1976, 13, 1111

Die erfindungsgemässen Substanzen besitzen eine hervorragende antibakterielle Wirkung gegen verschiedene pathogene Bakterienstämme. Beispiele für solche Stämme sind Escherichia, Klebsiella, Enterobacter, Serrattia, Acinetobacter, Pseudomonas, Staphylococcus, Streptococcus, Bacteroides, Clostridium und Peptococcus.

Zur Bestimmung der antibakteriellen Wirksamkeit wurden die MHK-Werte (minimale Hemmkonzentrations-Werte) in vitro sowie die $ED_{50}$-Werte in vivo gemessen. Als Vergleichssubstanzen dienten Ciprofloxacin (die bevorzugte Substanz der EP 78 362), Pefloxacin (die bevorzugte Substanz der DE-OS 2 843 066) sowie Enoxacin (die bevorzugte Substanz der EP 9 425). Die Überlegenheit der erfindungsgemässen Substanzen zeigt sich insbesondere gegenüber grampositiven Bakterien und bei oraler Applikation, da die Resorption der erfindungsgemäßen Verbindungen gegenüber Ciprofloxacin, Pefloxacin und Enoxacin deutlich verbessert ist.

Die Verbindungen der allgemeinen Formel I können als Heilmittel, z.B. in Form von pharmazeutischen Präparaten Verwendung finden, welche sie in Mischung mit einem für die enterale oder parenterale Applikation geeigneten pharmazeutischen, organischen oder anorganischen inerten Hilfs- und/oder Trägermaterial, wie z.B. pharmazeutisch unbedenklichen Lösungsmitteln, Gelatine, Gummi arabicum, Milchzucker, Stärke, Magnesiumstearat, Talk, pflanzliche Öle, Polyalkylenglykole, Vaseline und dergleichen, enthalten.

Die pharmazeutischen Präparate können in fester Form, beispielsweise als Tabletten, Dragees, Suppositorien, Kapseln und dergleichen, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstoffe, wie Konservierungs-,Stabilisierungs- oder Emulgiermittel, Salze zur Veränderung des osmotischen Druckes und dergleichen. Insbesondere können pharmazeutische Präparate die erfindungsgemäß herstellbaren Verbindungen in Kombination mit anderen therapeutisch wertvollen Stoffen enthalten. Mit diesen können die erfindungsgemäß herstellbaren Verbindungen zusammen mit den oben angegebenen Hilfs- und/oder Trägerstoffen zu Kombinationspräparaten formuliert werden.

Beispiel A: (wird nicht beansprucht)

Zu einer Lösung von 1,5 g (4 mMol) des gemischten Anhydrids von 6,7-Difluor-1-(4-fluorphenyl)-1,4-dihydro-4-oxo-chinolin-3-carbonsäure und Difluorborsäure in 10 ml Dimethylsulfoxid werden 0,9 g (4,78 mMol) 2-(Phenylmethyl)-2,5-diazabicyclo (2.2.1) heptan und 0,4 g (4 mMol) Triethylamin zugegeben. Nachdem 4 Stunden bei Raumtemperatur gerührt wurde, wird die Reaktionslösung mit 150 ml Ether verdünnt. Es bildet sich ein Niederschlag, der abfiltriert wird. Der Niederschlag wird in einer Mischung aus 60 ml Dioxan und 90 ml 1,5 N NaOH-Lösung dispergiert und eine Stunde bei 100°C erhitzt. Die Lösung wird filtriert, um ungelöstes Material zu entfernen. Dann wird mit konzentrierter HCl neutralisiert. Der entstandene Niederschlag wird abfiltriert, mit destilliertem Wasser gewaschen und an der Luft getrocknet. Nach Umkristallisieren aus DMF werden 1,23 g 6-Fluor-1-(4-fluor-phenyl)-1,4-dihydro-4-oxo-7-(5-phenylmethyl-2,5-diaza-bicyclo(2.2.1)hept-2-yl)-chinolin-3-carbonsäure erhalten (Schmelzpunkt: 275–280°C unter Zersetzung)

Ausbeute: 1,23 g (63% der Theorie)

Beispiel 1:

15,0 g (37,36 mmol) des gemischten Anhydrids von 7-Chlor-6-fluor-1-(2,4-difluorphenyl)-1,4-dihydro-4-oxo-chinolin-3-carbonsäure und Difluorborsäure, hergestellt durch Erhitzen von 7-Chlor-6-fluor-1-(2,4-difluorphenyl)-1,4-dihydro-4-oxo-chinolin-3-carbonsäure in 60%-iger wässriger Fluorborsäure, 15,36 g (56 mmol) des Dihydrobromids von 2-Methyl-2,5-diaza-bicyclo(2.2.1)heptan und 31 ml (0,224 mol)Triethylamin werden zu 200 ml trockenem Dimethylsulfoxid gegeben und 6 Tage bei Raumtemperatur gerührt. Danach wird die Reaktionsmischung in 800 ml kaltes Wasser gegossen. Der sich bildende Niederschlag wird abfiltriert, mit Wasser gewaschen und dann in einem Gemisch aus 100 ml Dioxan und 150 ml 3 N Natronlauge eine Stunde zum Rückfluß erhitzt. Nach dem Abkühlen werden unter Rühren 500 ml konzentrierte Salzsäure zugegeben. Es wird eine Stunde nachgerührt, vom Unlöslichen abfiltriert und das klare gelbe Filtrat noch mehrmals mit Methylenchlorid extrahiert. Die wässrige Phase wird dann unter Eiskühlen durch Zugabe von festen Natriumhydroxyd auf dem pH-Wert 7,4 gebracht. Anschließend wird mit Chloroform extrahiert. Die Chloroformphase wird mit Natriumsulfat getrocknet und das Chloroform im Vakuum abdestilliert. Das so erhaltene dunkelbraune Öl gibt nach Kristallisation aus 450 ml Ethanol 3,15 g 6-Fluor-1-(2,4-difluorphenyl)-1,4-dihydro-7-(5-methyl-2,5-diaza-bicyclo-(2.2.1)-hept-2-yl)-4-oxo-chinolin-3-carbonsäure als Hydrat, Schmp. 215–220°C (Zersetzung).

Ausbeute: 3,15 g (18,8% der Theorie)

Beispiel 2:

Analog zu Beispiel A werden bei Verwendung von 5,56 g (13,85 mmol) des gemischten Anhydrids von 7-Chlor-6-fluor-1-(2,4-difluorphenyl)-1,4-dihydro-4-oxo-chinolin-3-carbonsäure und Difluorborsäure, 5,82 g (16,62 mmol) 2-Phenylmethyl-2,5-diazabicyclo(2.2.1)heptan Dihydrobromid und 11,58 ml (83 mmol) Triäthylamin in 50 ml trockenem Dimethylsulfoxid 2,73 g von 6-Fluor-1-(2,4-difluorphenyl)-1,4-dihydro-4-oxo-7-(5-phenylmethyl-2,5-diaza-bicyclo(2.2.1) hept-2-yl)chinolin-3-carbonsäure mit einem Schmp. von 233–238°C (Zersetzung) nach Umkristallisation aus Toluol erhalten.

Ausbeute: 2,73 g (39% der Theorie)

Beispiel 3:

2,57 g (5,08 mmol) von 6-Fluor-1-(2,4-difluorphenyl)-1,4-dihydro-4-oxo-7-(5-phenylmethyl-2,5-diaza-bicyclo(2.2.1)hept-2-yl)chinolin-3-carbonsäure wird in 170 ml absolutem Ethanol, welches 18 g Salzsäure enthält, gelöst und mit 2,1 g 10%iger Palladium-Aktivkohle versetzt. Es wird 2 Stunden lang bei 15 psi und 60°C hydriert. Nach Entfernen des Katalysators wird unter vermindertem Druck eingedampft. Der erhaltene Feststoff wird in 80 ml Wasser aufgenommen und ein pH-Wert von 7 eingestellt. Die erhaltene Suspension wird 20 Minuten auf 60°C erhitzt. Nach Abkühlen wird der Niederschlag abfiltriert. Nach Umkristallisieren aus Dimethylformamid erhält man 1,53 g 7-(2,5-Diazabicyclo(2.2.1)hept-2-yl)-6-fluor-1-(2,4-difluorphenyl)-1,4-dihydro-4-oxo-chinolin-3-carbonsäure als Hydrat, Schmp.: 198–204°C (Zersetzung).

Ausbeute: 1,53 g (69,5% der Theorie)

Beispiel 4:

0,5 g (1,2 mmol) 7-(2,5-Diazabicyclo(2.2.1)hept-2-yl)-6-fluor-1-(2,4-difluorphenyl)-1,4-dihydro-4-oxo-chinolin-3-carbonsäure werden in 20 ml Dimethylformamid suspendiert und mit 3 ml gemischtem Anhydrid aus Essigsäure und Ameisensäure versetzt. Nach 90-minütigem Rühren bei Raumtemperatur wird das Lösungsmittel unter vermindertem Druck abdestilliert. Der ölige Rückstand wird nach Verrühren mit Äther fest und kann abfiltriert werden. Umkristallisation aus einer Mischung von 20 ml Dimethylformamid und 5 ml Ligroin gibt 0,24 g 7-(5-Formyl-2,5-diazabicyclo(2.2.1)hept-2-yl)-6-fluor-1-(2,4-difluorphenyl)-1,4-dihydro-4-oxo-chinolin-3-carbonsäure. Schmp.: 252–258°C (Zersetzung).

Ausbeute: 0,24 g (45,3% der Theorie)

Beispiel 5:

188 mg (0,434 mmol) von 7-(2,5-Diazabicyclo(2.2.1)hept-2-yl)-6-fluor-1-(2,4-difluorphenyl)-1,4-dihydro-4-oxo-chinolin-3-carbonsäuremonohydrat (Beispiel 3) werden in 20 ml trockenem Dimethylformamid suspendiert, mit 96 mg (0,95 mmol) wasserfreiem Kaliumhydrogencarbonat und 92 mg (0,48 mmol) 4-Brommethyl-5-methyl-1,3-dioxolen-2-on versetzt und zehn Stunden bei Raumtemperatur gerührt. Anschließend wird auf eine Mischung von 150 ml Ethylacetat und 50 ml Wasser gegossen und die Phasen getrennt. Die organische Phase wird fünf mal mit je 30 ml Wasser gewaschen, über Natriumsulfat getrocknet, filtriert und bei vermindertem Druck bei Raumtemperatur eingeengt. Umkristallisation aus Ethylacetat gibt 63 mg 1-(2,4-Difluorphenyl)-6-fluor-1,4-dihydro-7-(5((5-methyl-2-oxo-1,3-dioxolen-4-yl)methyl)-2,5-diazabicyclo(2.2.1)hept-2-yl)-4-oxo-chinolin-3-carbonsäure.

Schmp.: 133–138°C (Zersetzung)

Ausbeute: 63 mg (27,5% der Theorie)

<u>Beispiel B:</u>

Bestimmung der antibakteriellen in vitro-Eigenschaften:

Die antibakteriellen Eigenschaften der Verbindungen der Beispiele 1 und 3 wurden im MHK-Test (Agarverdünnungstest, Medium nach Müller-Hinton) gegenüber verschiedenen gram-positiven und gram-negativen Bakterienstämmen bestimmt und mit Pefloxacin verglichen. Die Ergebnisse sind in der Tabelle zusammengefaßt.

Tabelle

| Organismus | MHK (mcg/ML) | | |
|---|---|---|---|
| | Verb. 1 | Verb. 3 | PF |
| Escherichia coli 1 | 0.015 | 0.008 | 0.060 |
| Escherichia coli 2 | 0.015 | 0.008 | 0.060 |
| Escherichia coli 3 | 0.060 | 0.030 | 0.060 |
| Escherichia coli 4 | 0.030 | 0.008 | 0.030 |
| Klebsiella pneumoniae 1 | 0.060 | 0.030 | 0.120 |
| Klebsiella pneumoniae 2 | 0.060 | 0.060 | 0.120 |
| Klebsiella pneumoniae 3 | 0.120 | 0.060 | 0.250 |
| Enterobacter cloacae 1 | 0.060 | 0.060 | 0.120 |
| Enterobacter cloacae 2 | 0.030 | 0.015 | 0.060 |
| Enterobacter cloacae 3 | 0.060 | 0.030 | 0.250 |
| Serratia marcescens 1 | 0.120 | 0.060 | 0.060 |
| Serratia marcescens 2 | 4.000 | 4.000 | 4.000 |
| Serratia marcescens 3 | 0.500 | 0.250 | 0.250 |
| Morganella morganii 1 | 0.250 | 0.060 | 0.250 |
| Morganella morganii 2 | 0.250 | 0.120 | 0.030 |
| Morganella morganii 3 | 0.120 | 0.030 | 0.030 |
| Proteus rettergi | 0.060 | 0.030 | 0.120 |
| Providentia stuartii | 4.000 | 2.000 | 4.000 |
| Citrobacter diversus | 0.030 | 0.008 | 0.030 |
| Pseudomonas aeruginosa 1 | 1.000 | 0.500 | 4.000 |
| Pseudomonas aeruginosa 2 | 4.000 | 1.000 | 8.000 |
| Pseudomonas aeruginosa 3 | 0.500 | 0.250 | 2.000 |
| Staphylococcus aureus 1 | 0.030 | 0.250 | 0.250 |
| Staphylococcus aureus 2 | 0.015 | 0.120 | 0.250 |
| Staphylococcus aureus 3 | 0.500 | 0.250 | 2.000 |
| Staphylococcus aureus 4 | 0.030 | 0.250 | 0.250 |
| Streptococcus faecalis 1 | 0.500 | 0.500 | 4.000 |
| Streptococcus faecalis 2 | 0.500 | 0.500 | 2.000 |
| Streptococcus faecalis 3 | 0.500 | 0.500 | 4.000 |

PF: Pefloxacin

**Patentansprüche für die Vertragsstaaten: BE, CH, DE, FR, GB, IT, LI, NL, SE**

1. 4-Pyridonderivate der allgemeinen Formel

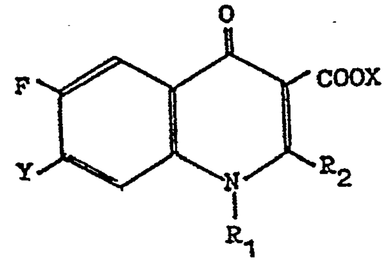

I

in der
$R_1$ 2,4-Difluorphenyl,
$R_2$ Wasserstoff,

X Wasserstoff oder eine physiologisch unbedenkliche Estergruppe und
Y eine Base der allgemeinen Formel

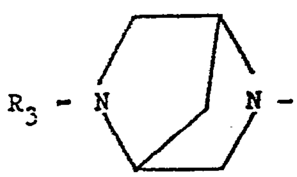

IIa oder

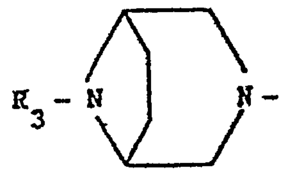

IIb

worin
$R_3$ für Wasserstoff, $(C_1–C_4)$-Alkyl, Benzyl, Formyl, Acetonyl oder einen Rest der allgemeinen Formel

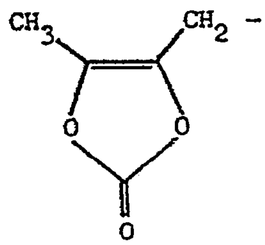

III

steht,
bedeuten, sowie Additionssalze von Verbindungen der allgemeinen Formel I mit pharmazeutisch unbedenklichen anorganischen oder organischen Säuren oder Basen und/oder deren Hydrate.

2. Verbindungen der allgemeinen Formel I nach Anspruch 1, worin $R_1$ 2,4-Difluorphenyl und Y eine Base der allgemeinen Formel II a, in der $R_3$ für Wasserstoff, Methyl oder Formyl steht, bedeutet.

3. 6-Fluor-1-(2,4-difluorphenyl)-1,4-dihydro-7-(5-methyl-2,5-diaza-bicyclo(2.2.1)hept-2-yl)-4-oxo-chinolin-3-carbonsäure.

4. 7-(2,5-Diazabicyclo(2.2.1)hept-2-yl)-6-fluor-1-(2,4-difluorphenyl)1,4-dihydro-4-oxo-chinolin-3-carbonsäure.

5. Verfahren zur Herstellung von Verbindungen der im Anspruch 1 definierten allgemeinen Formel I, dadurch gekennzeichnet, daß man

a) Verbindungen der allgemeinen Formel

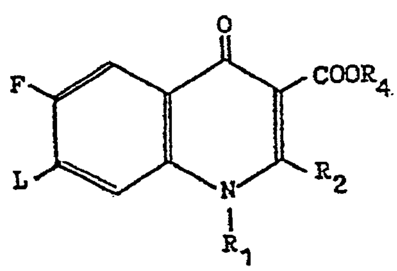

IV

in der $R_1$ und $R_2$ die in der allgemeinen Formel I angegebene Bedeutung haben, L eine nucleophile Abgangsgruppe wie Chlor oder Fluor, und $R_4$ Wasserstoff, $(C_1–C_4)$-Alkyl oder einen Borkomplex $-B(R_5R_6)$ darstellt, worin $R_5$ und $R_6$ gleich sind und Fluor oder einen Rest $-O(CO)-(C_1-C_4)$Alkyl bedeuten, mit Verbindungen der Formel

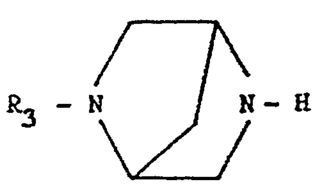

Va oder

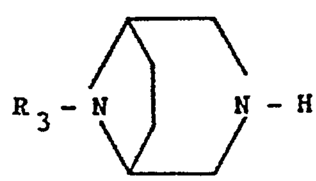

Vb

in der $R_3$ die in Formel I angegebene Bedeutung hat oder zusätzlich eine Aminoschutzgruppe bedeutet, umsetzt, worauf man

b) so erhaltene Verbindungen, in denen $R_4$ ($C_1$–$C_4$)-Alkyl oder die Gruppe $-B(R_5R_6)$ darstellt, durch Abspaltung von $-B(R_5R_6)$ oder von Alkyl in Verbindungen der Formel I, in denen X Wasserstoff bedeutet, überführt,

c) in erhaltenen Verbindungen, die in Position $R_3$ eine Aminoschutzgruppe tragen, diese abspaltet,

d) gegebenenfalls erhaltene Verbindungen der allgemeinen Formel I, in der $R_3$ Wasserstoff bedeutet, methyliert, benzyliert, formyliert oder mit Bromaceton oder einer Verbindung $R_7$Hal, in der $R_7$ den Rest der Formel III bedeutet, umsetzt und

e) gewünschtenfalls in Stufen a) bis d) erhaltene Verbindungen der allgemeinen Formel I in ihre pharmazeutisch verträglichen Additionssalze mit Säuren oder Basen und/oder in ihre physiologisch unbedenklichen Ester überführt.

6. Verfahren nach Anspruch 5, dadurch gekennzeichnet, daß man im Verfahrensschritt a) die Umsetzung von Verbindungen der allgemeinen Formel IV, in der $R_4$ für $-B(R_5R_6)$ steht, mit Verbindungen der Formel Va oder Vb bei Temperaturen von Raumtemperatur bis 50°C bei einer Dauer von 1–30 Stunden durchführt.

7. Pharmazeutische Präparate, enthaltend Verbindungen der allgemeinen Formel I in Kombination mit üblichen galenischen Hilfs- und/oder Trägerstoffen.

8. Pharmazeutische Präparate, enthaltend Verbindungen der allgemeinen Formel I in Kombination mit anderen pharmazeutischen Wirkstoffen sowie üblichen galenischen Hilfs- und/oder Trägerstoffen.

9. Verbindungen nach Anspruch 1 zur Verwendung als Wirkstoffe für Arzneimittel zur Behandlung und Prophylaxe von bakteriellen Infektionen.

**Patentansprüche für die Vertragsstaaten: AT, GR, ES**

1. Verfahren zur Herstellung von neuen 4-Pyridonderivaten der allgemeinen Formel

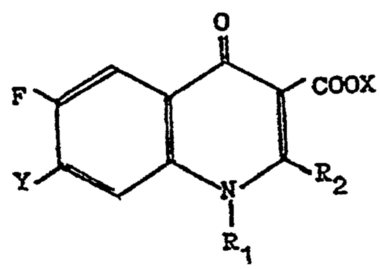

I

in der
$R_1$ 2,4-Difluorphenyl,
$R_2$ Wasserstoff,
X Wasserstoff oder eine physiologisch unbedenkliche Estergruppe und
Y eine Base der allgemeinen Formel

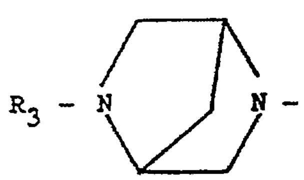

IIa

oder

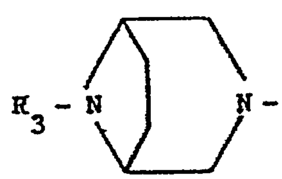

IIb

worin
$R_3$ für Wasserstoff, ($C_1$–$C_4$)-Alkyl, Benzyl, Formyl, Acetonyl oder einen Rest der allgemeinen Formel

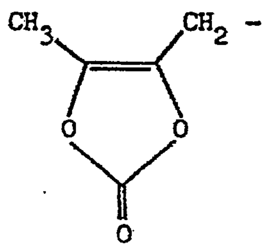

III

steht,

bedeuten, sowie von Additionssalzen von Verbindungen der allgemeinen Formel I mit pharmazeutisch unbedenklichen anorganischen oder organischen Säuren oder Basen und/oder deren Hydraten, dadurch gekennzeichnet, dass man

a) Verbindungen der allgemeinen Formel

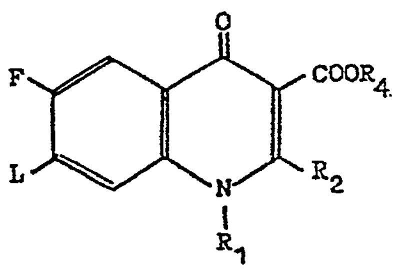

IV

in der $R_1$ und $R_2$ die in der allgemeinen Formel I angegebene Bedeutung haben, L eine nucleophile Abgangsgruppe wie Chlor oder Fluor, und $R_4$ Wasserstoff, $(C_1–C_4)$-Alkyl oder einen Borkomplex $–B(R_5R_6)$ darstellt, worin $R_5$ und $R_6$ gleich sind und Fluor oder einen Rest $–O(CO)–(C_1–C_4)$Alkyl bedeuten, mit Verbindungen der Formel

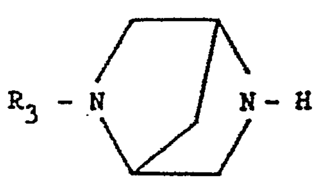

Va

oder

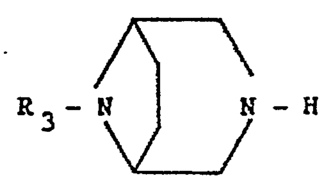

Vb

in der $R_3$ die in Formel I angegebene Bedeutung hat oder zusätzlich eine Aminoschutzgruppe bedeutet, umsetzt, worauf man

b) so erhaltene Verbindungen, in denen $R_4$ $(C_1–C_4)$-Alkyl oder die Gruppe $B(R_5R_6)$ darstellt, durch Abspaltung von $–B(R_5R_6)$ oder von Alkyl in Verbindungen der Formel I, in denen X Wasserstoff bedeutet, überführt,

c) in erhaltenen Verbindungen, die in Position $R_3$ eine Aminoschutzgruppe tragen, diese abspaltet,

d) gegebenenfalls erhaltene Verbindungen der allgemeinen Formel I, in der $R_3$ Wasserstoff bedeutet, methyliert, benzyliert, formyliert oder mit Bromaceton oder einer Verbindung $R_7$ Hal, in der $R_7$ den Rest der Formel III bedeutet, umsetzt und

e) gewünschtenfalls in Stufen a) bis d) erhaltene Verbindungen der allgemeinen Formel I in ihre pharmazeutisch verträglichen Additionssalze mit Säuren oder Basen und/oder in ihre physiologisch unbedenklichen Ester überführt.

2. Verfahren nach Anspruch 1, dadurch gekennzeichnet, daß man im Verfahrensschritt a) die Umsetzung von Verbindungen der allgemeinen Formel IV, in der $R_4$ für $–B(R_5R_6)$ steht, mit Verbindungen der Formel Va oder Vb bei Temperaturen von Raumtemperatur bis 50°C bei einer Dauer von 1–30 Stunden durchführt.

**Claims for the Contracting States: AT, GR ES**

1. Process for the preparation of new 4-pyridone derivatives of the general formula

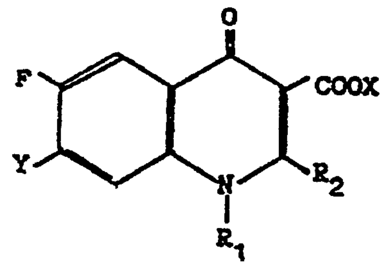

I

in which $R_1$ denotes 2,4-difluorophenyl, $R_2$ denotes hydrogen, X denotes hydrogen or a physiologically acceptable ester group and Y denotes a base of the general formula

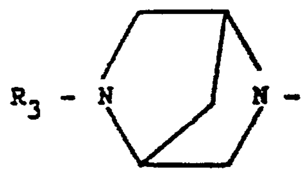

IIa

or

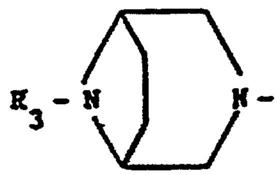

IIb

wherein $R_3$ represents hydrogen, $(C_1–C_4)$-alkyl, benzyl, formyl, acetonyl or a radical of the general formula

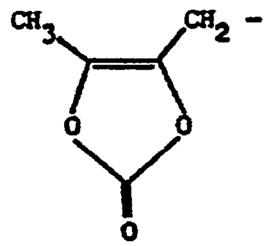

III

and of addition salts of compounds of the general formula I with pharmaceutically acceptable inorganic or organic acids or bases and/or their hydrates, characterized in that

a) compounds of the general formula

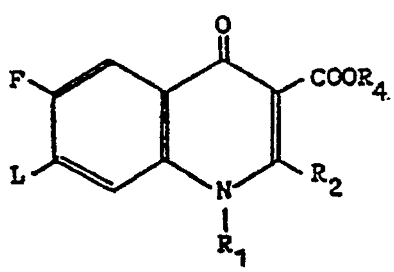

IV

in which $R_1$ and $R_2$ have the meaning given in the case of the general formula I, L represents a nucleophilic leaving group, such as chlorine or fluorine, and $R_4$ represents hydrogen, $(C_1–C_4)$-alkyl or a boron complex $–B(R_5R_6)$, wherein $R_5$ and $R_6$ are identical and denote fluorine or a $–O(CO)–(C_1–C_4)$-alkyl radical, are reacted with compounds of the formula

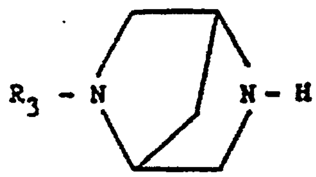

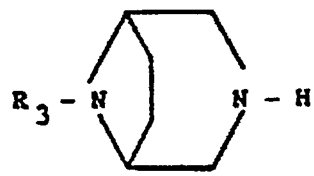

Va or Vb

in which $R_3$ has the meaning given in the case of formula I or additionally denotes an amino-protective group, after which

b) compounds thus obtained in which $R_4$ represents $(C_1-C_4)$-alkyl or the $-B(R_5R_6)$ group are converted into compounds of the formula I in which X denotes hydrogen by splitting off $-B(R_5R_6)$ or alkyl,

c) in resulting compounds which carry an amino-protective group in position $R_3$, this is split off,

d) if appropriate, resulting compounds of the general formula I in which $R_3$ denotes hydrogen are methylated, benzylated, formylated or reacted with bromoacetone or a compound $R_7Hal$, in which $R_7$ denotes the radical of the formula III, and

e) if desired, compounds of the general formula I obtained in stages a) to d) are converted into their pharmaceutically tolerated addition salts with acids or bases and/or into their physiologically acceptable esters.

2. Process according to claim 1, characterized in that in process step a), the reaction of compounds of the general formula IV in which $R_4$ represents $-B(R_5R_6)$ with compounds of the formula Va or Vb is carried out at temperatures from room temperature to 50°C over a period of 1–30 hours.

**Claims for the Contracting States: BE, CH, DE, FR, GB, IT, LI, NL, SE**

1. 4-Pyridone derivatives of the general formula

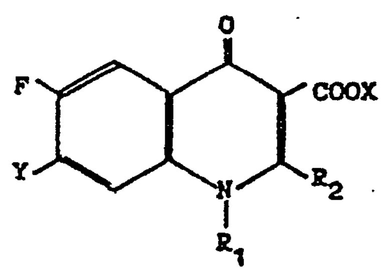

I

in which $R_1$ denotes 2,4-difluorophenyl, $R_2$ denotes hydrogen, X denotes hydrogen or a physiologically acceptable ester group and Y denotes a base of the general formula

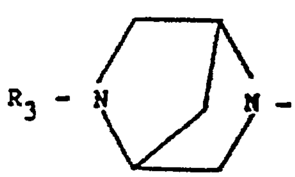

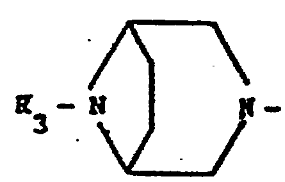

IIa or IIb

wherein $R_3$ represents hydrogen, $(C_1-C_4)$-alkyl, benzyl, formyl, acetonyl or a radical of the general formula

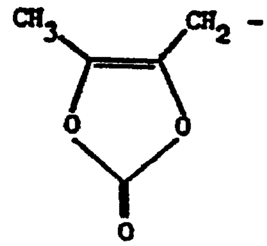

III

and addition salts of compounds of the general formula I with pharmaceutically acceptable inorganic or organic acids or bases and/or their hydrates.

2. Compounds of the general formula I according to claim 1, wherein $R_1$ denotes 2,4-difluorophenyl and Y denotes a base of the general formula IIa, in which $R_3$ represents hydrogen, methyl or formyl.

3. 6-Fluoro-1-(2,4-difluorophenyl)-1,4-dihydro-7-(5-methyl-2,5-diaza-bicyclo(2.2.1)hept-2-yl)-4-oxo-quinoline-3-carboxylic acid.

4. 7-(2,5-Diazabicyclo(2.2.1)hept-2-yl)-6-fluoro-1(2,4-difluorophenyl)-1,4-dihydro-4-oxo-quinoline-3-carboxylic acid.

5. Process for the preparation of compounds of the general formula I defined in claim 1, characterized in that

a) compounds of the general formula

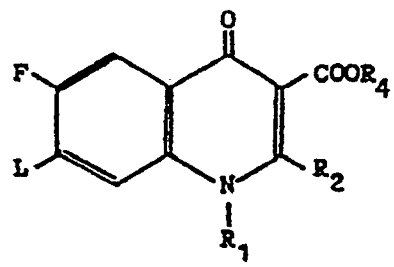

IV

in which $R_1$ and $R_2$ have the meaning given in the case of the general formula I, L represents a nucleophilic leaving group, such as chlorine or fluorine, and $R_4$ represents hydrogen, $(C_1–C_4)$-alkyl or a boron complex $–B(R_5,R_6)$, wherein $R_5$ and $R_6$ are identical and denote fluorine or a $–O(CO)–(C_1–C_4)$-alkyl radical, are reacted with compounds of the formula

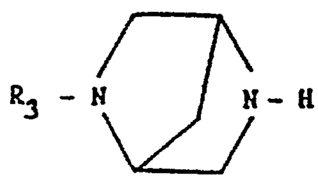

Va

or

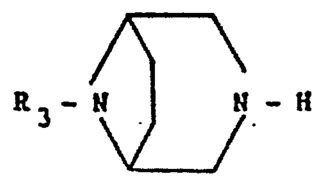

Vb

in which $R_3$ has the meaning given in the case of formula I or additionally denotes an amino-protective group, after which

b) compounds thus obtained in which $R_4$ represents $(C_1–C_4)$alkyl or the $–B(R_5R_6)$ group are converted into compounds of the formula I in which X denotes hydrogen by splitting off $–B(R_5R_6)$ or alkyl,

c) in resulting compounds which carry an amino-protective group in position $R_3$, this is split off,

d) if appropriate, resulting compounds of the general formula I in which $R_3$ denotes hydrogen are methylated, benzylated, formylated or reacted with bromoacetone or a compound $R_7$ Hal, in which $R_7$ denotes the radical of the formula III, and

e) if desired, compounds of the general formula I obtained in stages a) to d) are converted into their pharmaceutically tolerated addition salts with acids or bases and/or into their physiologically acceptable esters.

6. Process according to claim 5, characterized in that in process step a), the reaction of compounds of the general formula IV in which $R_4$ represents $–B(R_5R_6)$ with compounds of the formula Va or Vb is carried out at temperatures from room temperature to 50°C over a period of 1–30 hours.

7. Pharmaceutical preparations containing compounds of the general formula I in combination with customary galenical auxiliaries and/or excipients.

8. Pharmaceutical preparations containing compounds of the general formula I in combination with other pharmaceutical active compounds and customary galenical auxiliaries and/or excipients.

9. Compounds according to claim 1 for use as active compounds for medicaments for the treatment and prophylaxis of bacterial infections.

**Revendications pour les Etats contractants: AT, GR, ES**

1. Procédé de préparation de nouveaux dérivés 4-pyridone de formule générale I:

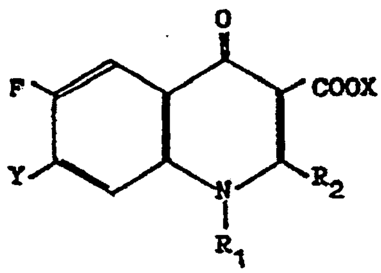

I

dans laquelle
$R_1$ représente le 2,4-difluorophényle,
$R_2$ l'hydrogène,
X l'hydrogène ou un groupe ester sans inconvénient physiologique,
Y une base de formule générale:

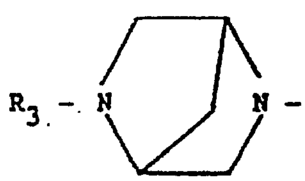

IIa

ou

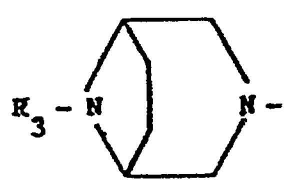

IIb

dans laquelle
$R_3$ designe l'hydrogène, un groupe alkyle de 1 à 4 atomes de carbone, benzyle, formyle, acétonyle ou un reste de formule générale III:

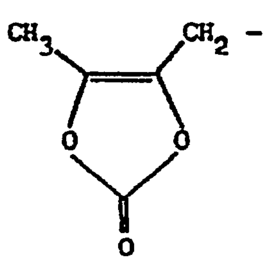

III

ainsi que les sels d'addition des composés de formule générale I avec des acides organiques ou non organiques sans inconvénient pharmaceutique ou des bases et/ou leurs composés hydratés, caractérisé en ce qu'on opère:

(a) en faisant réagir les composés de formule générale IV

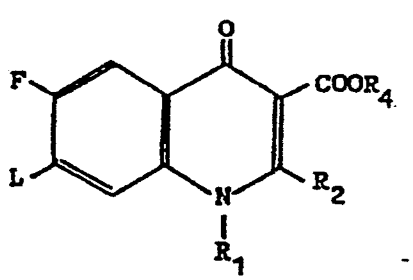

IV

dans laquelle $R_1$ et $R_2$ ont la signification indiquée dans la formule générale I, L un groupe mobile nucléophile tel le chlore ou le fluor, et $R_4$ l'hydrogène, un groupe alkyle de 1 à 4 atomes de carbone, ou

bien un complexe de bore $-B(R_5R_6)$ où $R_5$ et $R_6$ sont identiques et signifient le fluor ou un reste $-O(CO)-(C_1-C_4)$alkyle avec des composés de formule:

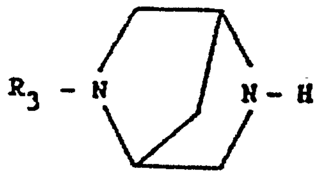

Va

ou

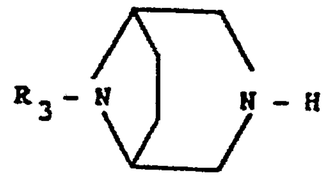

Vb

dans lesquelles $R_3$ a la signification indiquée dans la formule I ou additionnellement un groupe de protection amino,

(b) on convertit les composés ainsi obtenus dans lesquels $R_4$ représente un groupe alkyle-$(C_1-C_4)$ ou le groupe $-B(R_5R_6)$ par separation du groupe $-B(R_5R_6)$ ou alkyle en composés de formule I, dans lesquels X signifie l'hydrogène,

(c) dans les composés obtenus éliminer le groupe amino de protection en position $R_3$,

(d) on transforme les composés obtenus de formule générale I dans lesquels $R_3$ signifie l'hydrogène par méthylation, formylation ou par action du bromoacétone ou d'un composé $R_4$-Hal, dans lequel R désigne le groupe de formule III,

(e) le cas échéant transformer les composés obtenus dans les étapes (a) à (d) de formule générale I en leurs sels d'addition pharmaceutiquement admissibles avec des acides ou des bases et/ou en leurs esters sans inconvénients physiologiques.

2. Procédé selon la revendication 1, caractérisé en ce qu'on conduit, dans l'étape (a) du procédé, la réaction des composés de formule IV dans lesquels $R_4$ désigne le groupe $-B(R_5R_6)$, avec les composés de formule Va ou Vb à une température comprise entre la temperature ordinaire et 50°C, pendant 1 à 30 heures.

**Revendications pour les Etats contractants: BE, CH, DE, FR, GB, IT, LI, NL, SE**

1. Dérivé 4-pyridone de formule générale I:

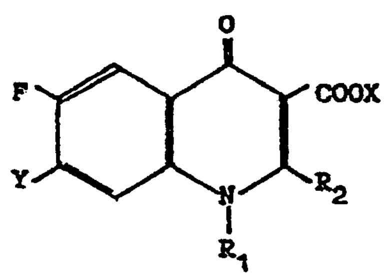

I

dans laquelle
$R_1$ représente le 2,4-difluorophényle,
$R_2$ l'hydrogène,
X l'hydrogène ou un groupe ester sans inconvénient physiologique,
Y une base de formule générale:

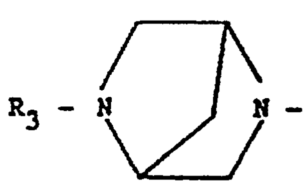

IIa

ou

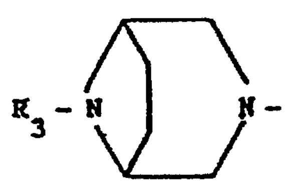

IIb

dans laquelle
$R_3$ désigne l'hydrogène, un groupe alkyle de 1 à 4 atomes de carbone, benzyle, formyle, acétonyle ou un reste de formule générale III:

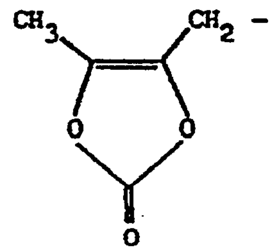

III

ainsi que les sels d'addition des composés de formule générale I avec des acides organiques ou non organiques sans inconvénient pharmaceutique ou des bases et/ou leurs composés hydratés.

2. Composés de formule générale I selon la revendication 1 dans lesquels $R_1$ représente le 2,4-difluorophényl et Y une base de formule générale IIa dans laquelle R signifie l'hydrogène, un groupe méthyle ou formyle.

3. Acide 6-fluoro-1-(2,4-difluorophényl)-1,4-dihydro-7-(5-méthyl-2,5-diazabicyclo(2.2.1)hept-2-yl)-4-oxo-quinoléine-3-carboxylique.

4. Acide 7-(2,5-diazabicyclo(2.2.1)hept-2-yl)-6-fluoro-1-(2,4-difluorophényl)-1,4-dihydro-4-oxo-quinoléine-3-carboxylique.

5. Procédé de préparation de composés selon la revendication 1, de formule générale I, caractérisé en ce qu'on opère:

(a) en faisant réagir les composés de formule générale IV

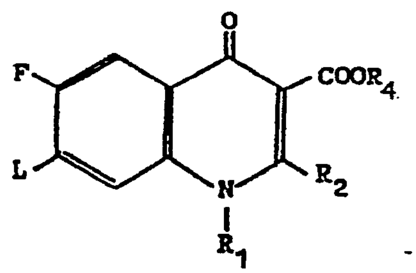

IV

dans laquelle $R_1$ et $R_2$ ont la signification indiquée dans la formule générale I, L un groupe mobile nucleophile tel le chlore ou le fluor, et $R_4$ l'hydrogène, un groupe alkyle de 1 à 4 atomes de carbone, ou bien un complexe de bore $-B(R_5R_6)$ où $R_5$ et $R_6$ sont identiques et signifient le fluor ou un reste (O(CO)-($C_1$–$C_4$)alkyle, avec des composés de formule:

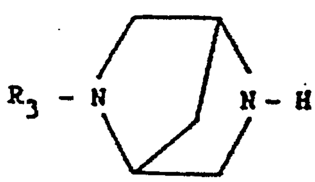

Va

ou

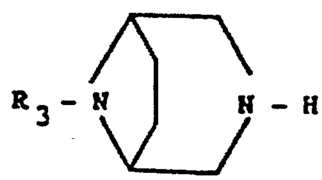

Vb

dans lesquelles $R_3$ a la signification indiquée dans la formule I ou additionnellement un groupe de protection amino,

(b) on convertit les composés ainsi obtenus dans lesquels $R_4$ représente un groupe alkyle $-(C_1–C_4)$ ou le groupe $-B(R_5R_6)$ par séparation du groupe $-B(R_5R_6)$ ou alkyle en composés de formule I, dans lesquels X signifie l'hydrogène,

(c) dans les composés obtenus éliminer le groupe amino de protection en position $R_3$,

(d) on transforme les composés obtenus de formule générale I dans lesquels $R_3$ signifie l'hydrogène par méthylation, formylation ou par action du bromoacétone ou d'un composé $R_4$-Hal, dans lequel R désigne le groupe de formule III,

(e) le cas échéant transformer les composés obtenus dans les étapes (a) à (d) de formule générale I en leurs sels d'addition pharmaceutiquement admissibles avec des acides ou des bases et/ou en leurs esters sans inconvénients physiologiques.

6. Procédé selon la revendication 5, caractérisé en ce qu'on conduit, dans l'étape (a) du procédé, la réaction des composés de formule IV dans lesquels $R_4$ désigne le groupe $-B(R_5R_6)$ avec les composés de formule Va ou Vb à une température comprise entre la température ordinaire et 50°C pendant 1 à 30 heures.

7. Préparations pharmaceutiques renfermant les composés de formule générale I en combinaison avec des adjuvants et/ou substances vectrices usuelles galéniques.

8. Préparations pharmaceutiques renfermant des composés de formule générale I en combinaison avec d'autres principes actifs pharmaceutiques ainsi qu'avec des adjuvants et substances vectrices galeniques usuelles.

9. Composés selon la revendication 1, utilisés comme principe actif dans les médicaments pour le traitement et la prophylaxie des infections bactériennes.